# EUROPÄISCHE PATENTSCHRIFT

(11) **EP 1 248 603 B1**
(45) Veröffentlichungstag und Bekanntmachung des Hinweises auf die Patenterteilung: **13.10.2004**
(21) Anmeldenummer: 01909624.7
(22) Anmeldetag: 15.01.2001
(51) Int. Cl.: A61K 31/00

(54) **VERWENDUNG SPEZIELLER VERBINDUNGEN ZUR PROPHYLAXE UND THERAPIE VON HEPATITIS C**
USE OF PARTICULAR COMPOUNDS FOR PROPHYLAXIS AND TREATMENT OF HEPATITIS C
UTILISATION DE COMPOSES SPECIAUX POUR LA PROPHYLAXIE ET LA THERAPIE DE L'HEPATITE C

(30) Priorität: 17.01.2000 DE 10001729
(43) Veröffentlichungstag der Anmeldung: 16.10.2002
(73) Patentinhaber: Morphochem AG, 81379 München (DE)
(72) Erfinder: WEBER, Lutz, 82110 Germering (DE); NERDINGER, Sven, 82008 Unterhaching (DE); CAPPI, Michael, W., 81369 München (DE); BEHNKE, Dirk, 82152 Planegg (DE)
(74) Vertreter: Forstmeyer, Dietmar, Dr. rer. nat., Dipl.-Chem.
(86) Internationale Anmeldenummer: PCT/EP2001/000390
(87) Internationale Veröffentlichungsnummer: WO 2001/052824

(56) Entgegenhaltungen:
- EP-A- 0 173 262
- EP-A- 0 288 962
- WO-A-94/02125
- WO-A-98/19670
- FR-A- 2 502 008
- FÖRTH W. ET AL: 'Allgemeine und spezielle Pharmakologie und Toxikologie', 1996, SPEKTRUM AKADEMISCHER VERLAG, HEIDELBERG, DE
- AUTERHOFF H. ET AL: 'Lehrbuch der Pharmazeutischen Chemie', 1991, WISSENSCHAFTLICHE VERLAGSGESELLSCHAFT, STUTTGART, DE

## Beschreibung

Die vorliegende Erfindung betrifft die Verwendung spezieller Verbindungen zur Prophylaxe und Therapie von Hepatitis C.

Hepatitis C ist eine chronische Virusinfektion von epidemischem Ausmaß. 400 Millionen Menschen, oder jeder Fünfzehnte weltweit und etwa 800 000 Deutsche leiden an dieser Krankheit; pro Jahr gibt es in Deutschland circa 40 000 neue Fälle.

60-80% der Infektionen mit dem Hepatitis C Virus (HCV) werden chronisch. Im Verlauf von 10-20 Jahren entwickeln 20- 30% der Patienten eine Leberzirrhose, 20-25 Jahre nach Infektion treten gehäuft Leberzellkarzinome auf. Es wird zur Zeit angenommen, daß etwa 20 % aller Betroffenen lebensbedrohliche Leber-Zustände entwickeln werden. Die Zahl der Todesopfer durch Hepatitis C hat sich in den letzten zehn Jahren verzehnfacht; Hepatitis C ist heute eine der zehn häufigsten Todesursachen in Deutschland. Bis heute ist die medikamentöse Behandlung von Hepatitis C nur ansatzweise gelungen.

WO 98/19670 offenbart eine Methode und Zusammensetzungen zur Behandlung einer Hepatitis C Infektion. Allgemein umfassen die Zusammensetzungen wenigstens zwei Verbindungen, die aus der aus Nucleosid Analogen, Quinolon Antibiotika und anti-viralen Amantadin-Mitteln bestehenden Gruppe ausgewählt werden. Wahlweise können diese Zusammensetzungen oder Arzneimittelkombinationen ein Interferon enthalten, beispielsweise Interferon α.

Es war daher die Aufgabe der vorliegenden Erfindung, Wirkstoffe zur Verwendung bei der Prophylaxe bzw. Therapie von Hepatitis C bereitzustellen.

Diese Aufgabe wird durch die Verwendung einer Verbindung der Formel (I): 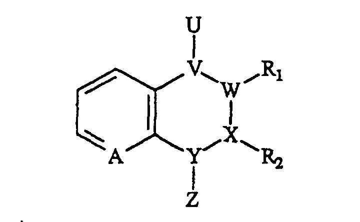 worin:
U-V-W(R₁)-X(R₂)-Y-Z die Formel RHN-C=CR₁-CR₂=C-OH, HO-C=CR₁-CR₂=C-OH oder O=C-CR₁=CR₂-C=O aufweist,
A ein N-Atom oder die Gruppe CH ist,
R ein H-Atom, eine C₁-C₆-Alkyl- oder eine Benzylgruppe ist,
R₁ ein H-Atom oder eine Methylgruppe ist, und
R₂ ein H-Atom oder eine Gruppe der Formel 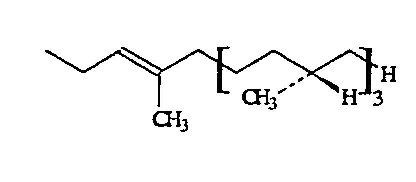 oder 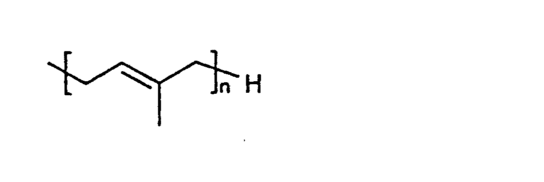 ist,
wobei n = 6, 7, 8 oder 9 ist,
zur Herstellung eines Medikaments zur Therapie oder Prophylaxe von Hepatitis C gelöst.
Vorzugsweise ist n = 7.
Vorzugsweise sind die Alkylreste eine Methyl-, Ethyl-, Propyl-, Isopropyl- oder Butylgruppe.

Gemäß einer Ausführungsform werden Verbindungen verwendet, bei denen Gruppe U-V-W(R₁)-X(R₂)-Y-Z die Formel RHN-C=CR₁-CR₂=C-OH 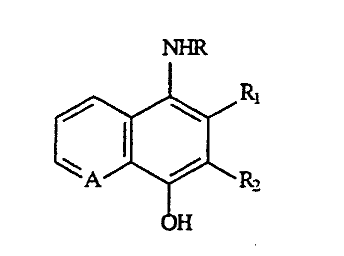 die Formel HO-C=CR₁-CR₂=C-OH: 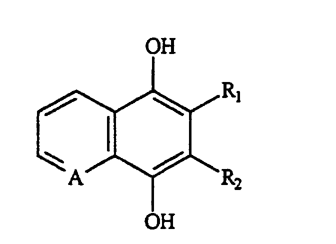 oder die Formel O=C-CR₁=CR₂-C=O aufweist:

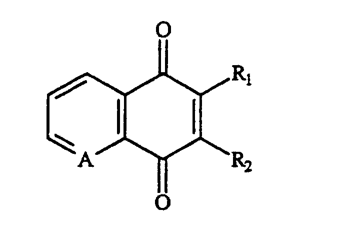

Vorzugsweise ist A eine CH-Gruppe: 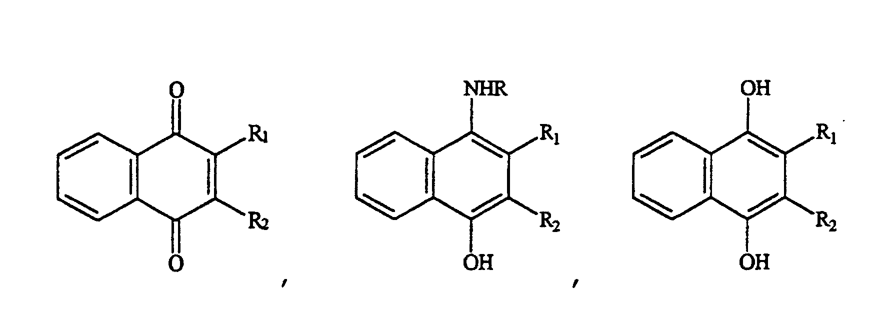 oder ein N-Atom:

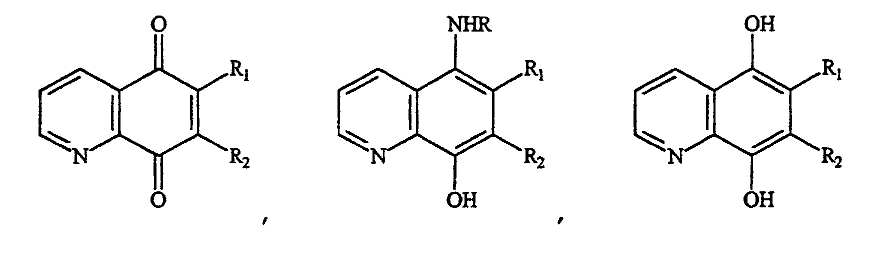

Gemäß einer besonders bevorzugten Ausführungsform ist R ein H-Atom oder eine Benzylgruppe.

Besonders bevorzugt wird die Verwendung von Verbindungen der Formeln: 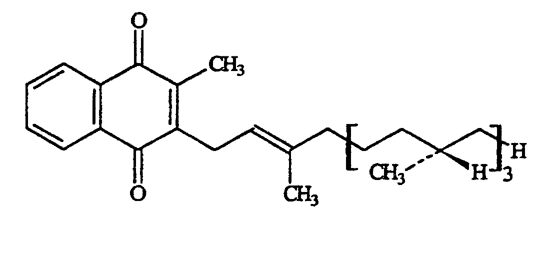 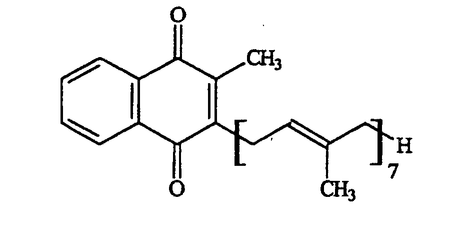 oder

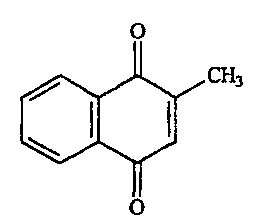

Derartige Verbindungen weisen eine besonders gute Wirkung bei der Prophylaxe oder Bekämpfung (Therapie) von Hepatis C (Viren) auf.

Zum Beispiel können die Verbindungen oral, parenteral, transdermal, intranasal, transmucosal, systemisch, subkutan, intravasculär, intramuskulär, topisch, rektal, intravaginal, verabreicht werden und Z.B. als Kapsel, Tablette, Pastille, Lutschbonbon, Spray, Nasenspray, Zäpfchen, Suppositorium, Injektionspräparat, Einlauf, Salbe, Creme oder Pflaster formuliert werden.

Auch können die Verbindungen als Präparate mit retardierter Freisetzung formuliert werden.
Die erfindungsgemäßen Wirkstoffe können als Prophylaktika oder Therapeutika in der Human- und Tiermedizin eingesetzt werden.

Dabei können sie lokal oder systemisch verwendet werden. Unter systemischer Applikation versteht man z.B. eine intravenöse, intrapleurale, intraperitoneale, rectale, orale, Applikation oder die Spülung von Körperhöhlen und Harnblase. Unter lokaler Applikation versteht man z.B. die subkutane, intrakutane, intratumorale, peritumorale Applikation, z.B. in Form von Injektionslösungen, Injektionssuspensionen, Cremes, Lotionen, Gelen und Salben.

Die erfindungsgemäß eingesetzten Wirkstoffe besitzen bei systemischer und bei lokaler Applikation eine dosisabhängige HCV bekämpfende Wirkung. Die Dosis der erfindungsgemäßen Wirkstoffe liegt bei therapeutischem Einsatz pro Tag in der Größenordnung von 0,01 bis 100 mg/kg Körpergewicht, vorzugsweise von 2 bis 40 mg/kg Körpergewicht. In individuellen Fällen kann die Dosierung größer oder kleiner sein als oben angegeben.

Die erfindungsgemäß verwendeten Wirkstoffe können in bekannter Weise - dem individuellen Krankheitsbild entsprechend - in einer Formulierung angewendet werden, wie Pflaster, Salben, Pasten, Cremes, lösliche Pulver, Emulsionen, Puder, Suspensionen und Injektionslösungen.

Die erfindungsgemäß verwendeten Wirkstoffe können beispielhaft in einer Injektionslösung gegebenenfalls unter Zuhilfenahme von Lösungsvermittlern in verdünnten physiologisch verträglichen Basen gelöst und durch Zugabe physiologisch verträglicher Säuren in eine injizierbare Form von pH 6 bis 8, insbesondere 6.9 bis 7.5 gebracht werden.

Beispielhafte physiologisch verträgliche Basen können Hydroxide, Hydrogencarbonate, Carbonate der Alkali- und Erdalkalimetalle, insbesondere von Kalium, Natrium und Calcium sein.

Beispielhafte physiologisch verträgliche Säuren können Milchsäure, Citronensäure, Weinsäure, Oxalsäure, Äpfelsäure, Essigsäure, Ameisensäure, Benzoesäure, Salicylsäure, Salzsäure, Schwefelsäure oder Phosphorsäure sein.

In der Formulierung der erfindungsgemäß verwendeten Wirkstoffe - die allein oder zu mehreren eingesetzt werden können - können Hilfsstoffe beigemischt werden. Solche nichttoxischen und pharmazeutisch geeigneten Hilfsstoffe können feste, halbfeste oder flüssige Trägerstoffe, Emulgier- oder Dispergiermitel sein. Die Konzentration der erfindungsgemäßen Wirkstoffe liegt zwischen 1 und 90 Gew.%, vorzugsweise 5 bis 50 Gew.%.

Die Dosierungseinheiten der erfindungsgemäß verwendeten Wirkstoffe können beispielsweise bestehen aus 1, 2, 3 oder 4 Einzeldosen oder 1/2, 1/3 oder 1/4 einer Einzeldosis. Eine Einzeldosis enthält vorzugsweise die Menge an Wirkstoff, die bei einer Applikation verabreicht wird und die gewöhnlich einer ganzen, einer halben oder einem Drittel oder gar einem Viertel einer Tagesdosis entspricht.

Cremes, Pasten, Salben und Gele können neben dem oder den Wirkstoffen übliche dem Fachmann bekannte Trägerstoffe, beispielsweise Wachse, Paraffine, Stärken, pflanzliche und tierische Fette, Cellulosederivate, Traganth, Kieselsäure, Talkum, Zinkoxid, Bentonite, Silicone, Polyäthylenglycole enthalten.

Sprays und Puder können neben dem oder den Wirkstoffen übliche dem Fachmann bekannte Trägerstoffe, wie Milchzucker, Talkum, Kieselsäure, Aluminiumhydroxid, Calciumsilikat oder Polyamidpulver oder Gemische davon enthalten. Sprays können zusätzlich Treibmittel, beispielsweise Fluorchlorkohlenwasserstoffe enthalten.

Suppositorien können neben dem oder den Wirkstoffen übliche dem Fachmann bekannte Trägerstoffe, wie Polyäthylenglycole, Fette oder Gemische davon enthalten.

Lokale Applikation der erfindungsgemäß verwendeten Wirkstoffe kann durch Mikromaschinen erfolgen.

Die Wirkstoffe können zur Erzielung besserer lokal-relevanter Wirkstoffkonzentrationen und zur besseren Verträglichkeit in Liposome verpackt werden.

Soweit für die Behandlung der Erkrankung oder für das Allgemeinbefinden des Patienten oder seiner Angehörigen von Nutzen können gleichzeitig oder in zeitlicher Versetzung Kombinationen mit anderen dem Patienten dienlichen Wirkstoffen verabreicht werden.

Die erfindungsgemäß verwendeten Verbindungen können in an sich üblicher Weise hergestellt werden.

### Beispiele:

Die IC₅₀-Werte wurden analog zu dem in: M. Taliani et al., Analytical Biochemistry, 240 (1996) 60-67 beschriebenen NS3 Proteinase-Assay bestimmt. Die Auswertung erfolgte mit Hilfe des Programms "GraFit 4" der Firma Erithacus Software Ltd. (Staines, Middlesex, UK).

### Beispiel 1: Menadion

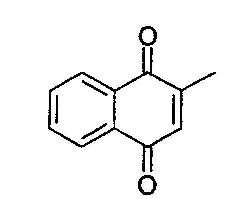

IC₅₀ = 45 µM

### Beispiel 2: 1,4-Naphthochinon

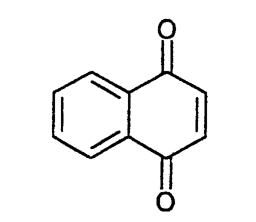

IC₅₀ = 20 µM

### Beispiel 3: 1-Amino-4-naphthol

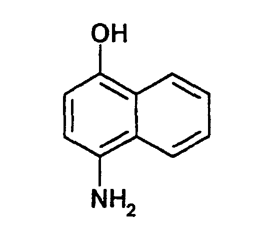

IC₅₀ = 13 µM

### Beispiel 4: 1-Benzylamino-4-naphthol

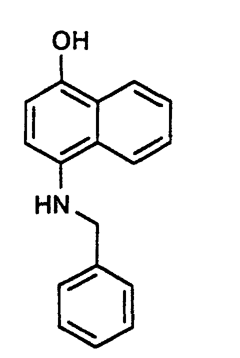

IC₅₀ = 25 µM

### Beispiel 5: 5-Amino-8-hydroxychinolin

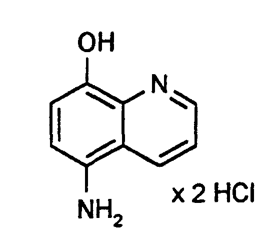

IC₅₀ = 51 µM

## Patentansprüche

1. Verwendung einer Verbindung der Formel (I): 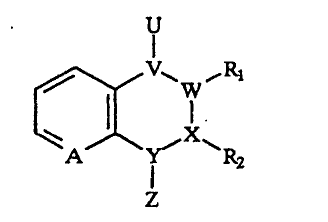 worin:
U-V-W(R₁)-X(R₂)-Y-Z die Formel RHN-C=CR₁-CR₂=C-OH, HO-C=CR₁-CR₂=C-OH oder O=C-CR₁=CR₂-C=O aufweist,
A ein N-Atom oder die Gruppe CH ist,
R ein H-Atom, eine C₁-C₆-Alkyl- oder eine Benzylgruppe ist,
R₁ ein H-Atom oder eine Methylgruppe ist, und
R₂ ein H-Atom oder eine Gruppe der Formel 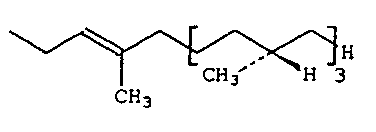 oder 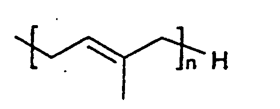
ist,
wobei n = 6, 7, 8 oder 9 ist,
Zur Herstellung eines Medikaments
zur Therapie oder Prophylaxe von Hepatitis C.

2. Verwendung nach Anspruch 1, **dadurch gekennzeichnet, daß** U-V-W(R₁)-X(R₂)-Y-Z die Formel RHN-C=CR₁-CR₂=C-OH aufweist:

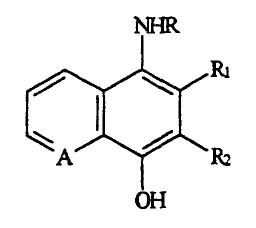

3. Verwendung nach Anspruch 1, **dadurch gekennzeichnet, daß** U-V-W(R₁)-X(R₂)-Y-Z die Formel HO-C=CR₁-CR₂=C-OH aufweist:

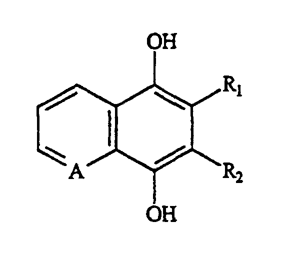

4. Verwendung nach Anspruch 1, **dadurch gekennzeichnet, daß** U-V-W(R₁)-X(R₂)-Y-Z die Formel O=C-CR₁=CR₂-C=O aufweist:

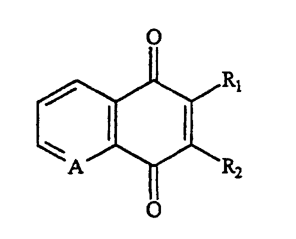

5. Verwendung nach einem der vorstehenden Ansprüche, **dadurch gekennzeichnet, daß** A eine CH-Gruppe ist: 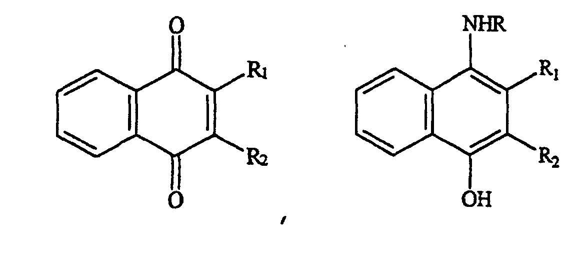 oder

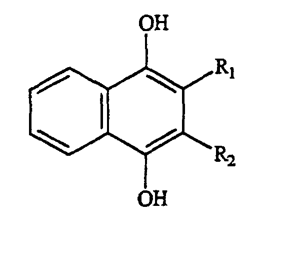

6. Verwendung nach einem der Ansprüche 1 bis 4, **dadurch gekennzeichnet, daß** A ein N-Atom ist: 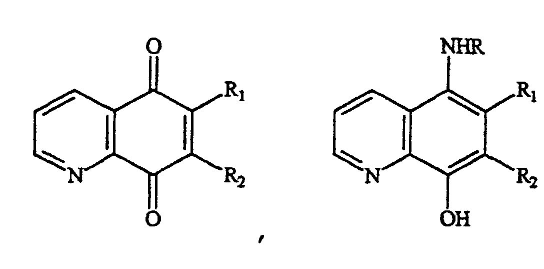 oder

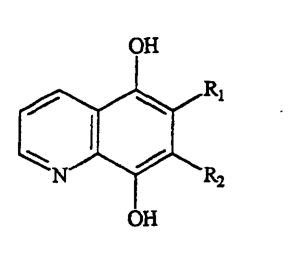

7. Verwendung nach einem der vorstehenden Ansprüche, **dadurch gekennzeichnet, daß** R ein H-Atom ist.

8. Verwendung nach einem der Ansprüche 1 bis 6, **dadurch gekennzeichnet, daß** R eine Benzylgruppe ist.

9. Verwendung nach einem der vorstehenden Ansprüche, **dadurch gekennzeichnet, daß** die Verbindung die Formeln: 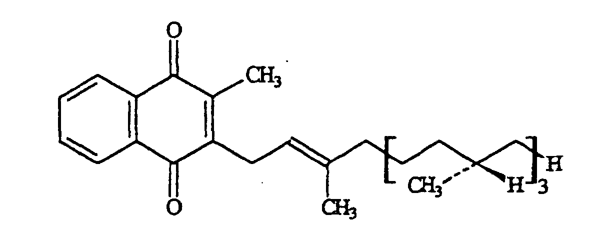 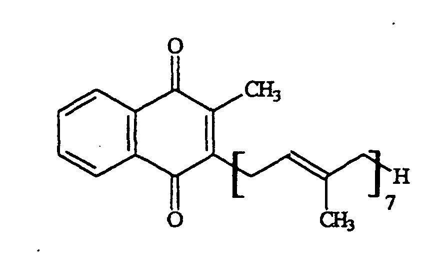 oder 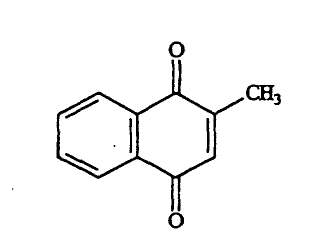 aufweist.

10. Verwendung nach einem der vorstehenden Ansprüche, **dadurch gekennzeichnet, daß** das Medikament oral, parenteral, transdermal, intranasal, transmucosal, systemisch, subkutan, intravasculär, intramuskulär, topisch, rektal, intravaginal, verabreicht wird.

11. Verwendung nach einem der vorstehenden Ansprüche, **dadurch gekennzeichnet, daß** die Verbindung als Kapsel, Tablette, Pastille, Lutschbonbon, Spray, Nasenspray, Zäpfchen, Suppositorium, Injektionspräparat, Einlauf, Salbe, Creme oder Pflaster formuliert wird.

12. Verwendung nach einem der vorstehenden Ansprüche, **dadurch gekennzeichnet, daß** die Verbindung als Präparat mit retardierter Freisetzung formuliert wird.

13. Verwendung nach einem der vorstehenden Ansprüche, **dadurch gekennzeichnet, daß** zusätzlich ein weiterer Wirkstoff eingesetzt wird.

## Claims

1. Use of a compound of formula (I): 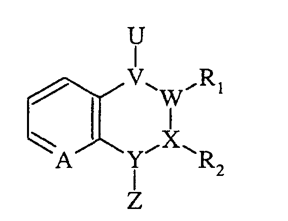 wherein:
U-V-W(R₁)-X(R₂)-Y-Z has the formula RHN-C=CR₁-CR₂=C-OH, HO-C=CR₁-CR₂=C-OH or O=C-CR₁=CR₂-C=O,
A is an N atom or the group CH,
R is an H atom, a C₁-C₆alkyl group or a benzyl group,
R₁ is an H atom or a methyl group, and
R₂ is an H atom or a group of formula 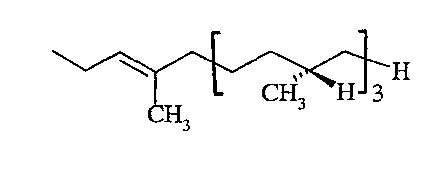 or 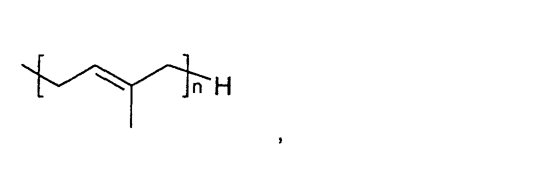 n being 6, 7, 8 or 9,
for the manufacture of a medicament for therapy or prophylaxis of hepatitis C.

2. Use according to claim 1, **characterised in that** U-V-W(R₁)-X(R₂)-Y-Z has the formula RHN-C=CR₁-CR₂=C-OH:

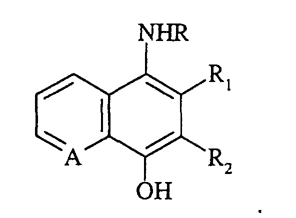

3. Use according to claim 1, **characterised in that** U-V-W(R₁)-X(R₂)-Y-Z has the formula HO-C=CR₁-CR₂=C-OH:

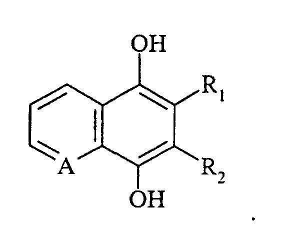

4. Use according to claim 1, **characterised in that** U-V-W(R₁)-X(R₂)-Y-Z has the formula O=C-CR₁=CR₂-C=O:

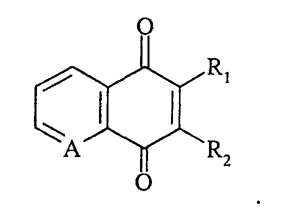

5. Use according to one of the preceding claims, **characterised in that** A is a CH group: 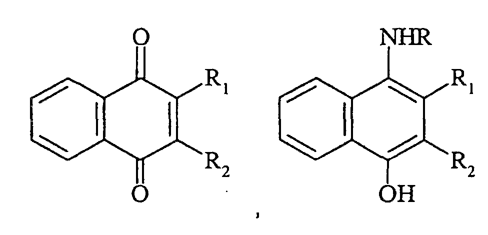 or

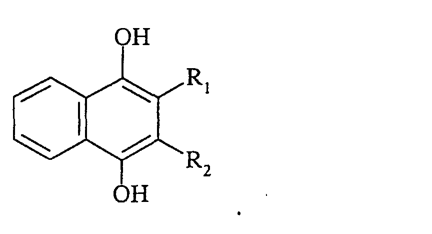

6. Use according to one of claims 1 to 4, **characterised in that** A is an N atom:

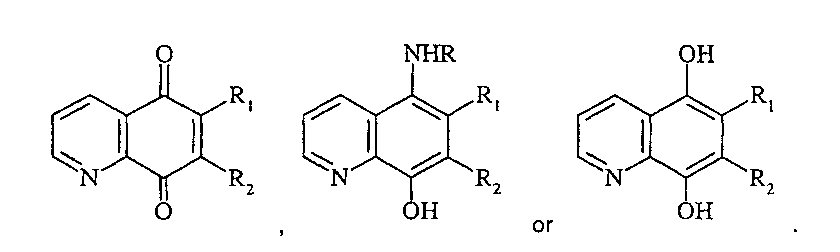

7. Use according to one of the preceding claims, **characterised in that** R is an H atom.

8. Use according to one of claims 1 to 6, **characterised in that** R is a benzyl group.

9. Use according to one of the preceding claims, **characterised in that** the compound has the formula:

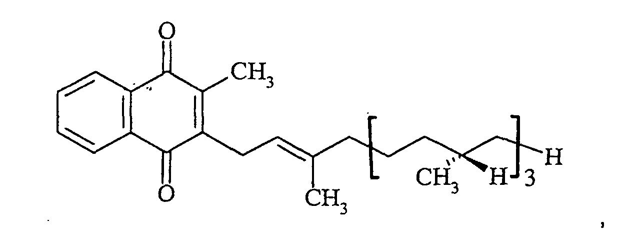

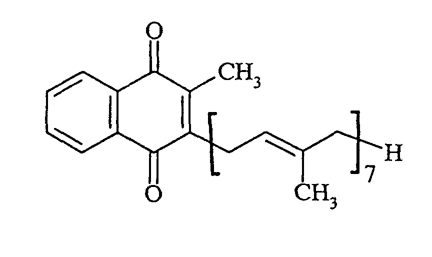

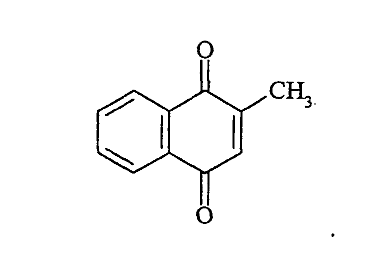

10. Use according to one of the preceding claims, **characterised in that** the medicament is administered orally, parenterally, transdermally, intranasally, transmucosally, systemically, subcutaneously, intravascularly, intramuscularly, topically, rectally or intravaginally.

11. Use according to one of the preceding claims, **characterised in that** the compound is formulated as a capsule, tablet, pastille, lozenge, spray, nasal spray, pessary, suppository, injection preparation, enema, ointment, cream or patch.

12. Use according to one of the preceding claims, **characterised in that** the compound is formulated as a delayed-release preparation.

13. Use according to one of the preceding claims, **characterised in that**, in addition, a further active ingredient is used.

## Revendications

1. Utilisation d'un composé de formule (I) : 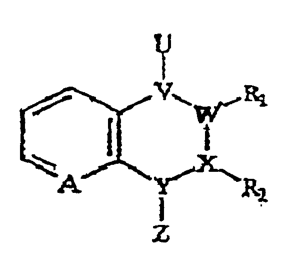 dans laquelle :
U-V-W(R₁)-X(R₂)-Y-Z présente la formule RHN-C=CR₁-CR₂=C-OH, HO-C=CR₁-CR₂=C-OH ou O=C-CR₁=CR₂-C=O,
A est un atome N ou le groupe CH,
R est un atome H, un groupe alkyle C₁-C₆ ou un groupe benzyle,
R₁ est un atome H ou un groupe méthyle et
R₂ est un atome H ou un groupe de formule 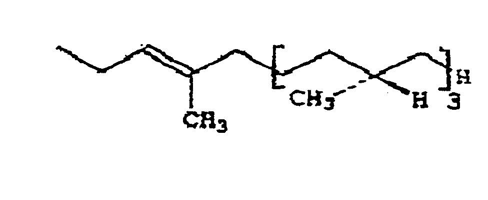 ou 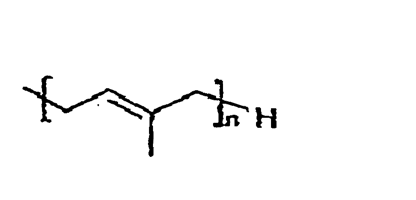
dans laquelle n = 6, 7, 8 ou 9,
pour la fabrication d'un médicament pour le traitement ou la prophylaxie de l'hépatite C.

2. Utilisation selon la revendication 1, **caractérisée en ce que** U-V-W(R₁)-X(R₂)-Y-Z présente la formule RHN-C=CR₁-CR₂=C-OH :

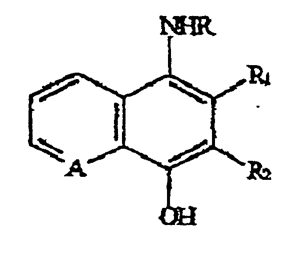

3. Utilisation selon la revendication 1, **caractérisée en ce que** U-V-W(R₁)-X(R₂)-Y-Z présente la formule HO-C=CR₁-CR₂=C-OH :

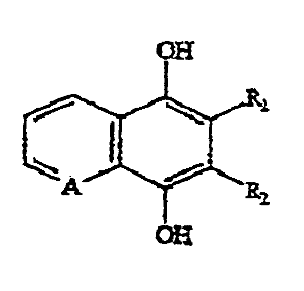

4. Utilisation selon la revendication 1, **caractérisée en ce que** U-V-W(R₁)-X(R₂)-Y-Z présente la formule O-C=CR₁-CR₂=C-O :

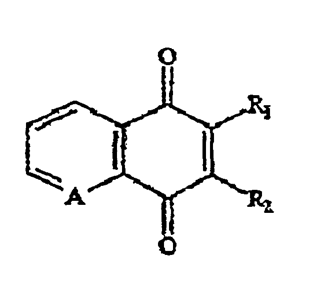

5. Utilisation selon l'une des revendications précédentes, **caractérisée en ce que** A est un groupe CH : 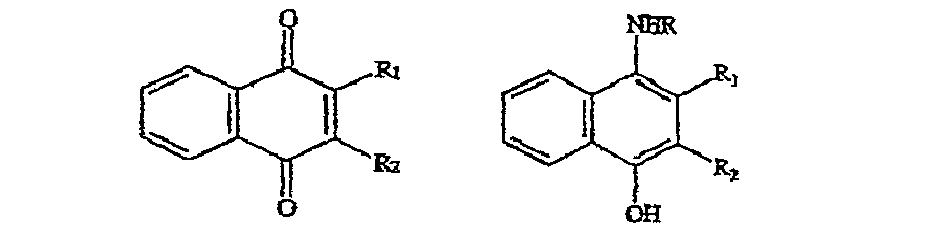 ou

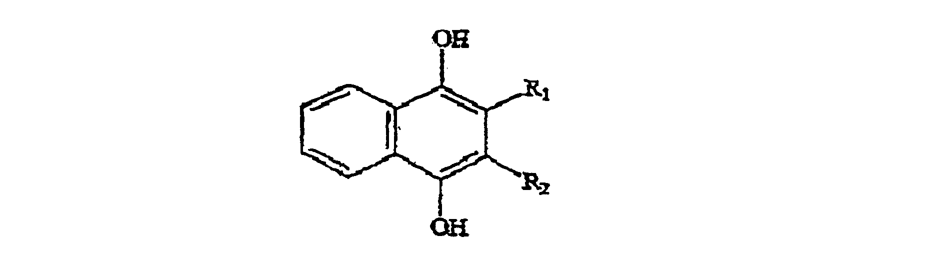

6. Utilisation selon l'une des revendications 1 à 4, **caractérisée en ce que** A est un atome N : 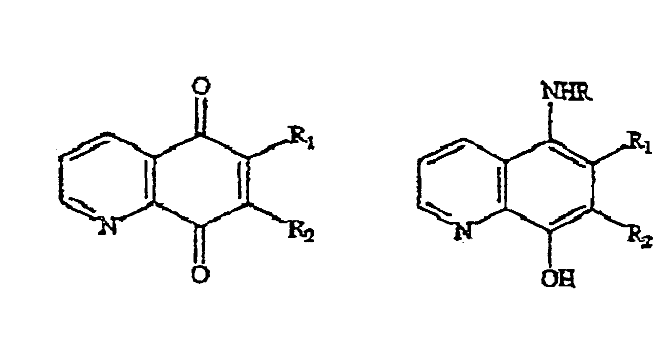 ou

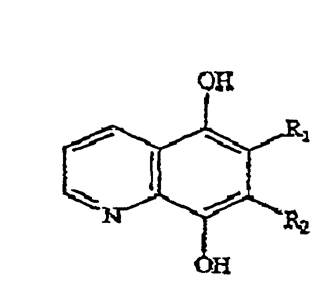

7. Utilisation selon l'une des revendications précédentes, **caractérisée en ce que** R est un atome H.

8. Utilisation selon l'une des revendications 1 à 6, **caractérisée en ce que** R est un groupe benzyle.

9. Utilisation selon l'une des revendications précédentes, **caractérisée en ce que** le composé présente les formules : 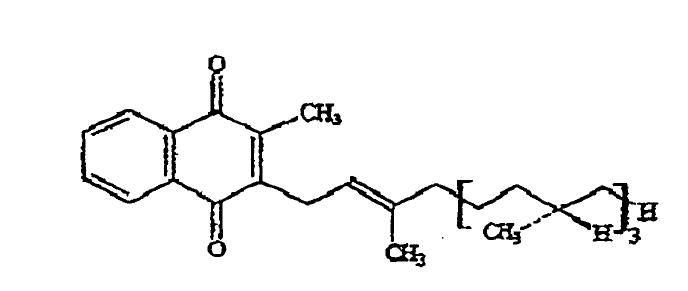 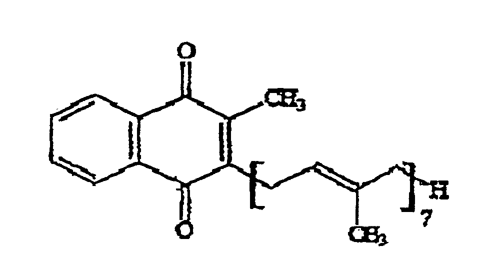 ou

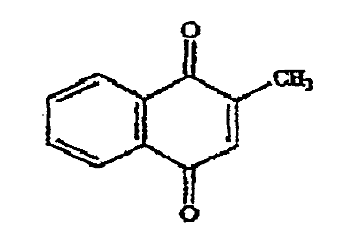

10. Utilisation selon l'une des revendications précédentes, **caractérisée en ce que** le médicament est administré par voie orale, parentérale, transdermique, intranasale, transmuqueuse, systémique, sous-cutanée, intravasculaire, intramusculaire, topique, rectale, intravaginale.

11. Utilisation selon l'une des revendications précédentes, **caractérisée en ce que** le composé est formulé en capsule, comprimé, pastille, comprimé à sucer, spray, spray nasal, suppositoire, préparation pour injection, lavement, onguent, crème ou pansement.

12. Utilisation selon l'une des revendications précédentes, **caractérisée en ce que** le composé est formulé comme une préparation à libération retardée.

13. Utilisation selon l'une des revendications précédentes, **caractérisée en ce qu'**en outre une autre substance active est utilisée.
